# EUROPÄISCHE PATENTSCHRIFT

(11) **EP 1 410 772 B1**
(45) Veröffentlichungstag und Bekanntmachung des Hinweises auf die Patenterteilung: **12.09.2007**
(21) Anmeldenummer: 03015073.4
(22) Anmeldetag: 03.07.2003
(51) Int. Cl.: A61F 2/52

(54) **Brustprothesen-Haftelement**
Attachment element for a breast prosthesis
Elément d'attachement pour une prothèse mammaire

(30) Priorität: 15.10.2002 DE 20215801 U
(43) Veröffentlichungstag der Anmeldung: 21.04.2004
(73) Patentinhaber: Thämert Orthopädische Hilfsmittel GmbH & Co. KG, 30938 Burgwedel/Grossburgwedel (DE)
(72) Erfinder: Schneider-Nieskens, Reinhold, 29223 Celle (DE)
(74) Vertreter: Siekmann, Gunnar

(56) Entgegenhaltungen:
- WO-A-94/16650
- DE-A- 19 754 144
- FR-A- 2 293 187
- US-A- 3 807 412

## Beschreibung

Die Erfindung betrifft eine Brustprothese mit einem der Rückseite der Brustprothese zugeordneten flächigen Haftelement, das auf einer Seite zur lösbaren Verbindung mit der Brustprothese und auf der anderen Seite zur lösbaren Verklebung mit der Haut einer Nutzerin ausgelegt ist. Mit einer solchen Haftschicht wird die Brustprothese auf der Haut einer Nutzerin lösbar verklebt. Derartige Brustprothesen werden auch als Haftprothesen bezeichnet.

Eine derartige Brustprothese ist aus der WO 94/16650 bekannt.

Derartige Brustprothesen mit einer Haftschicht sind insofern problematisch, als die Herstellung einer dauerhaft und zuverlässig klebenden Haftschicht, die auch nach mehrmaligem Gebrauch noch zuverlässig haftet, äußerst schwierig ist.

Der Erfindung liegt daher die Aufgabe zugrunde, eine Brustprothese der eingangs genannten Art mit einer dauerhaft zuverlässigen Haftschicht zu schaffen.

Die Lösung dieser Aufgabe erfolgt mit einer Brustprothese mit den Merkmalen des Patentanspruchs 1. Vorteilhafte Weiterbildungen der Erfindung sind in den Unteransprüchen beschrieben.

Bei einer Brustprothese mit einem der Rückseite der Brustprothese zugeordneten Haftelement, das auf einer Seite zur lösbaren Verbindung mit der Brustprothese und auf der anderen Seite zur lösbaren Verklebung mit der Haut einer Nutzerin ausgelegt ist, ist erfindungswesentlich, daß das Haftelement als ein flächiges Haftelement ausgebildet ist und daß das flächige Haftelement einen mit einem Stoff kaschierten Innenkörper aufweist. Auf diese Weise wird mit dem flächigen Haftelement, das auch als Haftpatte bezeichnet werden kann, ein Art Adapter zwischen Prothese und Körper zur Verfügung gestellt, wobei das Haftelement komplett abziehbar und damit austauschbar ist, so daß die Verwendungsdauer der eigentlichen Brustprothese wesentlich verlängert wird. Zudem wird mit dem Haftelement auch eine besonders gute formgerechte Anpassung bzw. ein Übergang auch von schalenförmigen Leichtprothesen zum Körper erreicht.

Das flächige Haftelement ist dabei als ein einziges durchgängiges Haftelement ausgebildet, das im wesentlichen die Rückseite der Brustprothese abdeckt. Denkbar sind Ausnehmungen oder ausgenommene Bereiche innerhalb des Haftelementes.

Das flächige Haftelement ist insbesondere auf der dem Körper zugewandten Seite bevorzugt mit einem Haftmaterial, insbesondere Haftsilikon oder einer Haftschicht aus Polyurethan oder Acrylaten oder anderen Polymeren, beschichtet. Derartige, aus dem medizinischen Bereich stammende Haftsilikone erlauben eine lösbare Verklebung am Körper bzw. haften am Körper und bilden eine auch aus medizinischer Sicht unbedenkliche Verbindung. Das flächige Haftelement ist bevorzugt auch auf der der Brustprothese zugewandten Seite mit Haftsilikon beschichtet, so daß auch auf dieser Seite eine Verklebung erfolgt. Dabei ist das flächige Haftelement mindestens auf der der Nutzerin zugewandten Seite bevorzugt auf der ganzen Fläche und bis zum bzw. bis einschließlich des Randes mit Haftsilikon beschichtet. Durch die Nutzung der gesamten Fläche des Haftelementes als Haftbereich wird eine besonders zuverlässige Verbindung erreicht. Durch die Ausbildung der Beschichtung bis zum Rand wird insbesondere auch vermieden, daß die Brustprothese am oberen Rand bei Belastung abrollt. Das Haftelement bildet eine durchgängige Fläche, die in Größe und Form der Rückseite der Brustprothese entspricht. Bevorzugt sind daher beide Seiten des flächigen Haftelementes mit dem Haftsilikon beschichtet. In einer alternativen vorteilhaften Ausgestaltung ist das flächige Haftelement der der Rückseite der Brustprothese zugewandten Seite mit einem Klettelement versehen. Dies können entweder der Klettfilz oder die Hakenelemente sein, die dann mit einem korrespondieren Klettelement auf der Rückseite der Brustprothese zusammenwirken.

Erfindungsgemäß weist das flächige Haftelement einen mit einem Stoff kaschierten Innenkörper auf. Dieser Stoff ist bevorzugt ein haftfähiges Textil, zum Beispiel ein Frotteestoff, auf den Haftmaterialien zuverlässig aufgebracht werden können. Der Innenkörper ist günstigerweise aus einem mit Polyurethan beschichteten Schaumstoff hergestellt. Ein solchermaßen aufgebautes flächiges Haftelement ist flexibel, haltbar und gut geeignet zur Aufnahme des klebrigen Haftsilikons. Im Gegensatz zu Haftstreifen wird durch die Verwendung eines kaschierten Innenkörpers ein Haftelement mit einer gewissen Tiefe oder einer insgesamt auch dreidimensionalen Ausbildung geschaffen. Ein solches Haftelement ist insgesamt auch leichter handhabbar.

Die Brustprothese ist bevorzugt als sogenannte Leichtprothese ausgebildet. Dazu werden in das Gel Mikrohohlkugeln eingemischt, die zu einer Gewichtsreduzierung führen. Weiterhin ist die Brustprothese günstigerweise schalenförmig ausgebildet, d.h., daß die Brustprothese auf der Rückseite im zentralen Bereich eine Ausnehmung oder Mulde aufweist, die einerseits zu einer Gewichtsreduzierung führt und andererseits auch die Anpassung und Verformung der Brustprothese positiv beeinflußt. Günstigerweise ist auf der Rückseite der Brustprothese ein ringförmiger flächiger Bereich vorgesehen. Dieser Bereich umgibt die schalenförmige Ausnehmung im zentralen Bereich der Rückseite der Brustprothese. In diesem ringförmigen Bereich, der günstigerweise ebenfalls mit Haftsilikon belegt ist, erfolgt die Verklebung mit dem flächigen Haftelement. Aufgrund der starken Klebewirkung zwischen der Rückseite der Brustprothese und dem Haftelement ist der nicht klebende mittlere schalenförmige Bereich weit weniger störend, als wenn eine direkte Verklebung der Brustprothese mit der Haut erfolgen würde. Weiterhin ist auf der Rückseite der Brustprothese im Randbereich eine gegenüber dem ringförmigen flächigen Bereich erhöhte Kante vorgesehen. Das flächige Haftelement ist korrespondierend dazu in einer bevorzugten Ausführungsform etwas kleiner als die Brustprothese ausgebildet, so daß das flächige Haftelement innen an der Kante der Rückseite der Brustprothese anliegt und auf diese Weise lagejustiert wird. Besonders bevorzugt ist es jedoch, das flächige Haftelement genauso groß wie die Rückseite der Brustprothese auszubilden, dabei jedoch den auf der Kante der Brustprothese liegenden Bereich etwas dünner, also gegenüber dem mittleren Bereich verjüngt, auszubilden, so daß das flächige Haftelement auf der der Nutzerin zugewandten Seite eine ebene, leicht konkav geformte Fläche bildet. Insgesamt muß jedoch hervorgehoben werden, daß es besonders bevorzugt ist, ausschließlich das Haftelement mit Haftmaterial zu beschichten und die Brustprothese nicht mit Haftmaterial zu beschichten, so daß diese auch als normale Brustprothese getragen werden kann. Grundsätzlich ist es jedoch möglich, die Verbindung zwischen Brustprothese und Haftelement durch die Aufbringung von Haftmaterial auch auf der Brustprothese oder auf Brustprothese und der der Brustprothese zugekehrten Seite des Haftelementes zu erreichen

Neben dem Vorteil der Auswechselbarkeit des Haftelementes wird insbesondere bei den schalenförmigen Brustprothesen auch erreicht, daß derartige Leichtprothesen überhaupt einsetzbar sind und ein Adapter zur Anpassung bzw. zum Ausgleich der muldenförmigen Schale gegeben ist. Bevorzugt wird bei der Leichtprothese ein mit Mikrohohlkugeln durchsetzter Gelkörper verwendet, der von einer Decksilikonschicht überzogen ist. Durch diese Ausbildungsform der Leichtprothese wirkt diese auch vergleichsweise stabil und ist besonders gut zur Verwendung mit dem Haftelement geeignet. In einer anderen bevorzugten Ausführungsform der Erfindung weist die Rückseite der Brustprothese bzw. die der Nutzerin oder dem Nutzer zugewandte Seite der Brustprothese eine umlaufende Kante oder einen erhöhten Rand aufweist und daß das Haftelement etwas kleiner als die Brustprothese ausgebildet ist und an der Innenseite des Randes der Brustprothese anliegt, also in die von dem Rand umgebende Fläche eingelegt werden kann. Besonders bevorzugt ist bei dieser Ausführungsform der Rand nicht ganz durchgehend ausgebildet, sondern weist im Bereich der oberen Spitze der Brustprothese eine Ausnehmung auf, wobei im Haftelement ein zu der Ausnehmung korrespondierender Vorsprung vorgesehen ist, so daß im Bereich der oberen Spitze das Haftelement bis unmittelbar oder bis 2 mm an den Rand der Brustprothese ausgeführt ist. Auf diese Weise wird die Haftsicherheit, insbesondere im Bereich der oberen Spitze, bei der bei Belastung der Rand sonst leicht abrollen kann, eine besonders gute Haftung und Verklebung geschaffen.

Nachfolgend wird die Erfindung anhand eines in der Zeichnung dargestellten bevorzugten Ausführungsbeispiels weiter erläutert. Im einzelnen zeigen die schematischen Darstellungen in:
- Fig. 1:: eine geschnittene Seitenansicht von Brustprothese und Haftelement;
- Fig. 2:: eine Draufsicht auf die Rückseite der Brustprothese;
- Fig. 3:: eine Draufsicht auf die der Rückseite der Brustprothese zugewandte Seite des Haftelementes;
- Fig. 4:: eine Draufsicht auf die Rückseite einer zweiten Ausführungsform einer erfindungsgemäßen Brustprothese;
- Fig. 5:: eine Draufsicht auf die der Rückseite der Brustprothese zugewandten Seite des Haftelementes der zweiten Ausführungsform der Erfindung;
und
- Fig. 6:: eine geschnittene Seitenansicht des Haftelementes entlang der Linie A-A in Fig. 5.

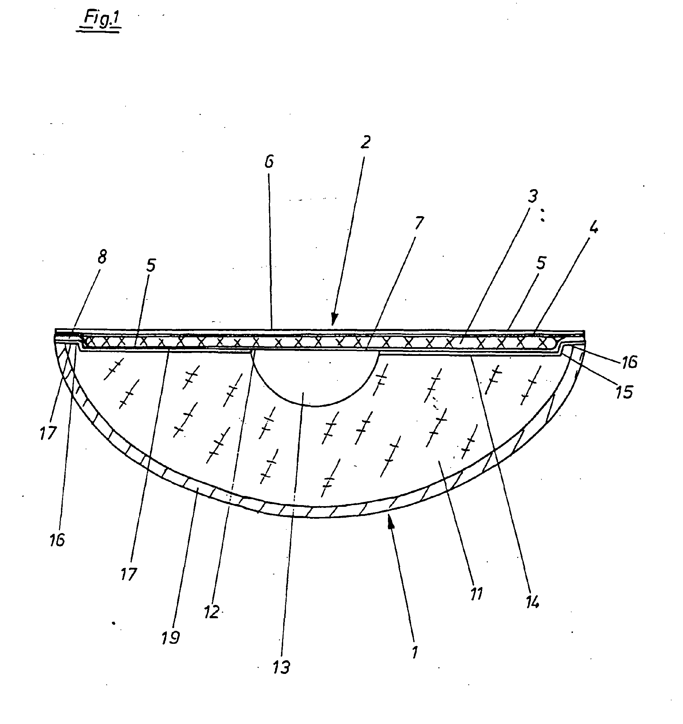

In Fig. 1 ist eine Querschnittsansicht durch Brustprothese 1 und das darauf aufgeklebte Haftelement 2 dargestellt. Die Brustprothese 1 wird im wesentlichen bestimmt durch den Gelkörper 11. Dieser ist mit einer Decksilikonschicht 19 überzogen, so wie es auch in der DE 197 54 144 A1 beschrieben ist. Der Gelkörper 11 besteht durch die Verwendung von Mikrohohlkugeln im wesentlichen aus sogenanntem Leichtsilikon. Die der Nutzerin zugewandten Rückseite 12 der Brustprothese 1 weist im zentralen Bereich eine Ausnehmung 13 auf, die der Brustprothese 1 einen schalenförmigen Charakter verleiht und auch zur Ausbildung der Brustprothese als Leichtprothese beiträgt. Um die Ausnehmung 13 herum ist ein flächiger Bereich 14 vorgesehen, der im Randbereich von einem nahezu vertikalen Abschnitt 15 begrenzt wird, der etwa 2mm bis 3mm hoch ist. Daran schließt sich eine im wesentlichen horizontale, jedoch leicht zum Gelkörper 11 hin geneigte Kante 16 an. Der flächige Bereich 14, der vertikale Abschnitt 15 und die Kante 16 sind mit Haftsilikon 17 belegt. Mit der Rückseite 12 der Brustprothese 1 ist das flächige Haftelement 2 verklebt. Dieses weist eine der Brustprothese 1 zugekehrte Seite 7 und eine der Nutzerin zugekehrte Seite 6 auf. Die Form und Abmessung des Haftelementes 2 wird im wesentlichen von einem Schaumstoffelement 3 bestimmt, das mit Polyurethan beschichtet ist und dann mit einem Textil 4, insbesondere einem Frotteestoff, kaschiert ist. Dieses Textil 4 ist mit Haftsilikon 5 durchtränkt bzw. überzogen, das auf der der Trägerin zugewandten Seite 6 mit der Haut der Trägerin und auf der der Rückseite der Brustprothese zugewandten Seite 7 mit der in dem flächigen Bereich 14 und den Randbereichen 15 und 16 vorgesehenen Haftsilikonschicht 17 verklebt. In den Randbereichen fehlt der aussteifende Schaumstoff 3, so daß sich dadurch im Randbereich 8 das gesamte Haftelement 2 verjüngt und dadurch die Geometrie der Brustprothese im Bereich der Kante 16 ausgleicht, so daß auf der der Trägerin zugewandten Seite 6 insgesamt eine leicht konkave durchgängige Fläche gebildet wird. Durch die Ausbildung der Kante 16 im Randbereich der Brustprothese 1 wird eine genaue und formschlüssige Ausrichtung des Haftelementes 2 auf der Brustprothese 1 ermöglicht.

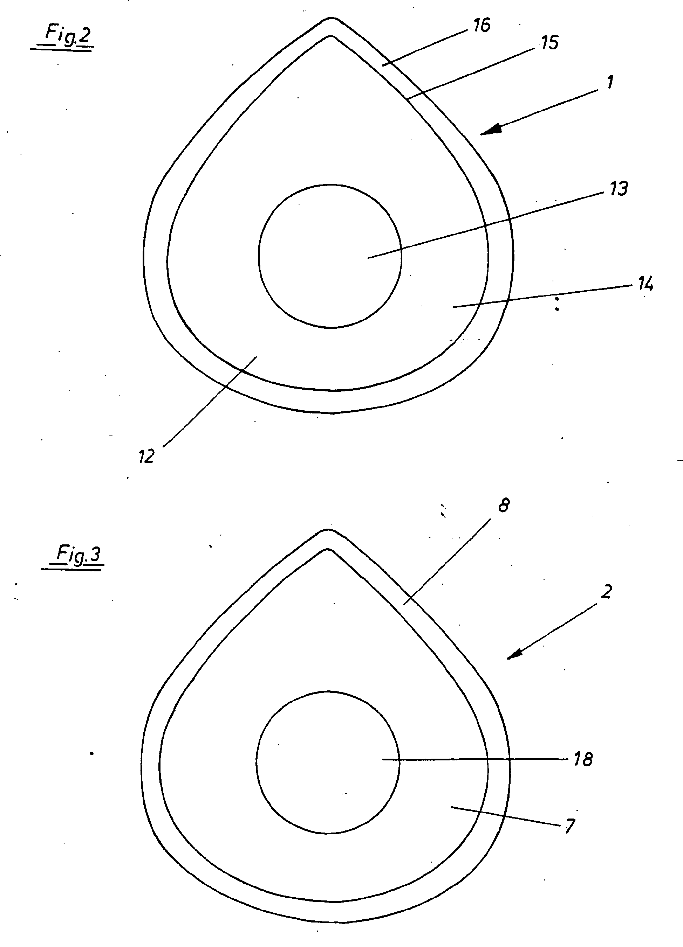

In Fig. 2 ist eine Draufsicht auf die Rückseite 12 der Brustprothese 1 dargestellt. Im zentralen Bereich der Brustprothese 1 ist eine Ausnehmung 13 vorgesehen, die insbesondere auch der Gewichtsersparnis dient. Um die Ausnehmung 13 herum ist ein flächiger Bereich 14 vorgesehen, an den sich nach außen der bereits in Fig. 1 beschriebene vertikale Abschnitt anschließt, der dann in die nahezu horizontale Kante 16 übergeht, die eine Breite von etwa 1cm hat. Sowohl der flächige Bereich 14 als auch der vertikale Abschnitt 15 als auch die Kante 16 sind bevorzugt mit Haftsilikon beschichtet. Die Ausnehmung 13 ist bevorzugt nicht mit Haftsilion beschichtet.

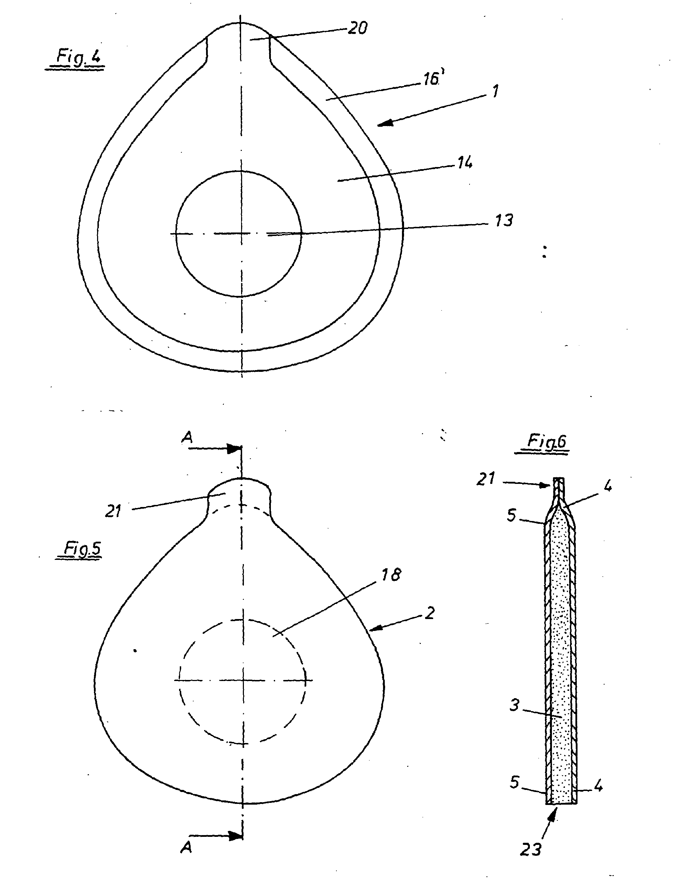

In Fig. 3 ist eine Draufsicht auf das Haftelement 2 von der der Brustprothese 1 zugewandten Seite 7 her dargestellt. Das Haftelement ist dabei im wesentlichen flächig ausgebildet, wobei der Randbereich 8 gegenüber dem übrigen Bereich nach hinten versetzt bzw. flacher ausgebildet ist, so daß auf diese Weise eine Anpassung an den vorstehenden Rand der Brustprothese 1 erfolgt. Das Haftelement 2 ist mit Haftmaterial, insbesondere Haftsilikon, überzogen. Auf der der Prothese zugewandten Seite ist der mit der Vertiefung der Prothese korrespondierende Teil nicht mit Haftmaterial belegt oder nach einer ursprünglich vollständigen Belegung mit Haftmaterial in diesem Bereich mit einer Folie abgedeckt, so daß die Ausnehmung 13 der Brustprothese 1 nicht an dem Haftelement 2 haftet. Dieser ausgesparte oder mit einer Folie bedeckte Bereich ist in der Fig. 3 mit 18 gekennzeichnet.

In den folgenden Figuren 4, 5 und 6 ist eine zweite Ausführungsform der erfindungsgemäßen Brustprothese beschrieben. In Fig. 4 ist, ähnlich wie in Fig. 2, eine Draufsicht auf die Rückseite der Brustprothese 1 dargestellt. Auch hier ist im zentralen Bereich eine Ausnehmung 13 vorgesehen, die insbesondere der Gewichtsersparnis dient. Um die Ausnehmung 13 herum ist ein flächiger Bereich 14 vorhanden. Der wesentliche Unterschied zur erstbeschriebenen Ausführungsform liegt in der Kante 16', die nicht ganz umlaufend ausgebildet ist, sondern im Bereich des oberen Ansatzes der Brustprothese eine Unterbrechung oder Ausnehmung 20 aufweist, in der sich der flächige Bereich 14 fortsetzt.

In Fig. 5 ist, ähnlich wie in Fig. 3, eine Draufsicht auf die der Rückseite der Brustprothese zugewandten Seite des Haftelementes dieser zweiten Ausführungsform dargestellt. Das Haftelement 2 ist in seinem Außenumfang um den Bereich der Kante 16' kleiner als das in Fig. 3 dargestellte Haftelement 2, da in dieser zweiten Ausführungsform das Haftelement 2 so ausgelegt ist, daß es vollständig innerhalb der Kante 16' auf dem flächigen Bereich 14 aufliegt, so daß dadurch insgesamt erreicht wird, daß die Prothese mit der Kante 16' auf der Haut liegt, statt wie in der ersten Ausführungsform auch in diesem Bereich mit dem Haftelement verklebt ist. Besonders wichtig für die Verklebung ist jedoch der obere Spitzenbereich der Prothese 1, da sich hier bei Belastung der Rand abrollen könnte. Hier wird das Haftelement 2 mit einem Vorsprung oder Ausläufer 21 ausgebildet, der größenmäßig an die Ausnehmung 20 in der Kante 16' angepaßt ist. Der Ausläufer reicht bis ca. 2 mm an den Rand heran.

In Fig. 6 ist ein Schnitt entlang der Linie A-A in Fig. 5 dargestellt. Der Aufbau des Haftelementes 2 entspricht dem auch in Fig. 1 dargestellten Aufbau. Der wesentliche Unterschied ist der, daß das Haftelement im Außenbereich eine gerade Abschlußkante 23 aufweist, die an das Innere der Kante 16' anstößt. Lediglich im Bereich des Ausläufers 21 verjüngt sich das Haftelement 2 zu dem schmalen Element, bestehend aus Haftsilikon und Textil, da der Schaumkern 3 nicht bis in den Ausläufer 21 hineingeführt ist.

## Patentansprüche

1. Brustprothese mit einem der Rückseite (12) der Brustprothese (1) zugeordneten flächigen Haftelement (2), das auf einer Seite (7) zur lösbaren Verbindung mit der Brustprothese (1) und auf der anderen Seite (6) zur lösbaren Verklebung mit der Haut einer Nutzerin ausgelegt ist,
**dadurch gekennzeichnet,**
**daß** das flächige Haftelement (2) einen mit einem Stoff (4) kaschierten Innenkörper (3) aufweist.

2. Brustprothese nach Anspruch 1, **dadurch gekennzeichnet, daß** das flächige Haftelement (2) mindestens teilweise mit einem Haftmaterial, insbesondere Haftsilikon (5), beschichtet ist.

3. Brustprothese nach einem der vorhergehenden Ansprüche, **dadurch gekennzeichnet, daß** das flächige Haftelement (2) mindestens auf der der Nutzerin zugewandten Seite (6) auf der gesamten Fläche und bis zum Rand mit dem Haftmaterial, insbesondere Haftsilikon (5), beschichtet ist.

4. Brustprothese nach einem der vorhergehenden Ansprüche, **dadurch gekennzeichnet, daß** das flächige Haftelement (2) auf der der Brustprothese zugewandten Seite (7) Klettelemente aufweist.

5. Brustprothese nach Anspruch 4, **dadurch gekennzeichnet, daß** der Innenkörper (3) von einem mit Polyurethan beschichteten Schaumstoff gebildet ist.

6. Brustprothese nach einem der vorhergehenden Ansprüche, **dadurch gekennzeichnet, daß** die Brustprothese schalenförmig ausgebildet ist.

7. Brustprothese nach einem der vorhergehenden Ansprüche, **dadurch gekennzeichnet, daß** auf der Rückseite (12) der Brustprothese (1) ein ringförmiger flächiger Bereich (14) vorgesehen ist.

8. Brustprothese nach einem der vorhergehenden Ansprüche, **dadurch gekennzeichnet, daß** auf der Rückseite (12) der Brustprothese (1) im Randbereich eine gegenüber dem ringförmigen flächigen Bereich (14) erhöhte Kante (16) vorgesehen ist.

9. Brustprothese nach einem der vorhergehenden Ansprüche, **dadurch gekennzeichnet, daß** das Haftelement (2) im äußeren Randbereich (8) dünner als im übrigen Bereich ausgebildet ist.

10. Brustprothese nach einem der vorhergehenden Ansprüche, **dadurch gekennzeichnet, daß** die Brustprothese als Leichtprothese ausgebildet ist.

11. Brustprothese nach einem der vorhergehenden Ansprüche, **dadurch gekennzeichnet, daß** das flächige Haftelement (2) als ein einziges durchgängiges Haftelement ausgebildet ist, das im wesentlichen die Rückseite der Brustprothese abdeckt.

## Claims

1. A breast prosthesis having a flat adhesive element (2) which is assigned to the back side (12) of the breast prosthesis (1) and is designed on one side (7) for detachable connection to the breast prosthesis (1) and on the other side (6) is designed for detachable gluing to the skin of the wearer,
**characterized in that**
the flat adhesive element (2) has an internal body (3) which is laminated to a fabric (4).

2. The breast prosthesis according to Claim 1,
**characterized in that**
the flat adhesive element (2) is at least partially coated with an adhesive material, in particular an adhesive silicone (5).

3. The breast prosthesis according to any one of the preceding claims,
**characterized in that**
the flat adhesive element (2) is coated with the adhesive material, in particular an adhesive silicone (5), over the entire area and up to the edge on at least the side (6) facing the wearer.

4. The breast prosthesis according to any one of the preceding claims,
**characterized in that**
the flat adhesive element (2) has Velcro-type closure elements on the side (7) facing the breast prosthesis.

5. The breast prosthesis according to Claim 4,
**characterized in that**
the internal body (3) is formed by a polyurethane-coated foam.

6. The breast prosthesis according to any one of the preceding claims,
**characterized in that**
the breast prosthesis is designed in the form of a shell.

7. The breast prosthesis according to any one of the preceding claims,
**characterized in that**
a ring-shaped flat area (14) is provided on the back side (12) of the breast prosthesis (1).

8. The breast prosthesis according to any one of the preceding claims,
**characterized in that**
an edge (16) that is elevated in comparison with the ring-shaped flat area (14) is provided in the edge area on the back side (12) of the breast prosthesis (1).

9. The breast prosthesis according to any one of the preceding claims,
**characterized in that**
the adhesive element (2) is designed to be thinner in the outer edge area (8) than in the remaining area.

10. The breast prosthesis according to any one of the preceding claims,
**characterized in that**
the breast prosthesis is designed as a lightweight prosthesis.

11. The breast prosthesis according to any one of the preceding claims,
**characterized in that**
the flat adhesive element (2) is designed as a single continuous adhesive element which covers essentially the back side of the breast prosthesis.

## Revendications

1. Prothèse de sein comportant un élément adhérent (2) associé à la face arrière (12) de la prothèse de sein (1) et qui est réalisé sur un côté (7) de manière à se raccorder de manière dissociable à la prothèse de sein (1) et sur l'autre côté (6) de manière à se coller de manière dissociable à la peau d'une utilisatrice,
**caractérisée en ce que**
l'élément adhérent surfacique (2) comporte un corps intérieur (3) plaqué d'un matériau (4).

2. Prothèse de sein selon la revendication 1, **caractérisée en ce que** l'élément adhérent surfacique (2) est revêtu du moins partiellement d'un matériau adhérent, notamment du silicone adhérent (5).

3. Prothèse de sein selon une des revendications précédentes, **caractérisée en ce que** l'élément adhérent surfacique (2) est revêtu, du moins du côté tourné vers l'utilisatrice (6), sur toute sa surface et jusqu'au bord, du matériau adhérent, notamment du silicone adhérent (5).

4. Prothèse de sein selon une des revendications précédentes, **caractérisée en ce que** l'élément adhérent surfacique (2) présente des éléments agrippants du côté tourné vers la prothèse de sein (7).

5. Prothèse de sein selon la revendication 4, **caractérisée en ce que** le corps intérieur (3) est constitué d'une mousse revêtue de polyuréthane.

6. Prothèse de sein selon une des revendications précédentes, **caractérisée en ce que** la prothèse de sein est réalisée en forme de coupe.

7. Prothèse de sein selon une des revendications précédentes, **caractérisée en ce que,** sur la face arrière (12) de la prothèse de sein (1), il est prévu une zone surfacique de forme annulaire (14).

8. Prothèse de sein selon une des revendications précédentes, **caractérisée en ce que,** sur la face arrière (12) de la prothèse de sein (1), il est prévu dans la zone périphérique un bord (16) surélevé par rapport à la zone surfacique de forme annulaire (14).

9. Prothèse de sein selon une des revendications précédentes, **caractérisée en ce que** l'élément adhérent (2) est réalisé plus mince dans sa zone périphérique extérieure (8) qu'ailleurs.

10. Prothèse de sein selon une des revendications précédentes, **caractérisée en ce que** la prothèse de sein est réalisée sous forme d'une prothèse légère.

11. Prothèse de sein selon une des revendications précédentes, **caractérisée en ce que** l'élément adhérent surfacique (2), est réalisé sous forme d'un unique élément adhérent continu qui recouvre sensiblement la face arrière de la prothèse de sein.
